# FASCICULE DE BREVET EUROPEEN

(11) **EP 0 625 039 B1**
(45) Date de publication et mention de la délivrance du brevet: **20.09.1995**
(21) Numéro de dépôt: 93914485.3
(22) Date de dépôt: 06.01.1993
(51) Int. Cl.: A61K 7/00

(54) **PREPARATION A USAGE CUTANE**
HAUTPFLEGEMITTEL
PREPARATION FOR SKIN USE

(30) Priorité: 16.01.1992 FR 9200619
(43) Date de publication de la demande: 23.11.1994
(73) Titulaire: COSMETICS DEVELOPMENT COMPANY LIMITED, Douglas, Isle of Man (GB)
(72) Inventeur: OLIVIER-TERRAS, Josette, F-01960 Peronnas (FR)
(74) Mandataire: Allen, William Guy Fairfax
(86) Numéro de dépôt international: FR9300006
(87) Numéro de publication internationale: WO9313742

(56) Documents cités:
- EP-A- 0 381 543
- EP-A- 0 447 318
- FR-A- 2 591 105
- FR-A- 2 664 164
- SEIFEN, ÖLE, FETTE, WACHSE vol. 117, no. 14, 5 Septembre 1991, AUGSBURG pages 514-517 R.A. BRUNKE ET AL." PRODUCTION AND IDENTIFICATION OF LIPOSOMES, SPHINGOSOMES AND NANOPARTICLES"
- SEIFEN, ÖLE, FETTE, WACHSE vol. 117, no. 5, 27 Mars 1991, AUGSBURG pages 180 - 186 A. HUC ET AL. 'A NEW SYSTEM OF ENCAPSULATION: COLLAGEN GLYCOSAMINOGLYCANS BASED CAPSULES'

## Description

L'invention concerne une préparation à usage cutané formée par un mélange d'excipients, de filtres solaires et de vecteurs médicamenteux, destinée à atténuer ou retarder l'apparition de taches mélaniques sur la peau.

Comme on le sait, l'expression "vecteurs médicamenteux" désigne des molécules creuses et de petite taille qui forment réservoirs pour des produits actifs qui se trouvent ainsi protégés et que l'on peut libérer ou relarguer le moment venu à l'endroit le mieux approprié (biodisponibilité). Les liposomes, décrits par exemple dans le document FR-A-2 315 991 correspondant au document US-A-4 217 344, sont les plus connus de ces vecteurs.

Les vecteurs médicamenteux encapsulant du béta-carotène sont connus pour leur action sur les hyperpigmentations cutanées d'origine mélanique, notamment les taches mélaniques photo-induites, les chloasmas, les mélasmas, les pigmentations iatrogènes ou cosmétiques majorées par le soleil, les pigmentations des frottements. Toutefois, cette action est assez longue, souvent partielle, voire aléatoire.

Dans le document FR-A-2 591 105, correspondant au document US-A-5 034 228, on a décrit une préparation à usage cutané à base de liposomes contenant, à titre d'agent actif un caroténoïde, tel qu'un caroténoïde, notamment du béta-carotène. Dans une forme de réalisation pratique (exemples 5 et 6), la suspension de liposomes contient également un filtre UV-A+B (sans autre précision). Cette préparation est utilisée pour le traitement de l'élastose solaire, la régénération de la peau ou comme agent cicatrisant. Malheureusement, ce type de préparation ne s'est guère développé, sans doute, par suite :
. d'une part, de la taille appréciable des liposomes, qui pour certaines applications cutanées, limitent la pénétration de la préparation dans les interstices cellulaires, notamment entre les cornéocytes ;
. d'autre part, de l'instabilité in-vitro et in-vivo dans le temps des liposomes, ce qui peut provoquer des relargages intempestifs du béta-carotène encapsulé, qui alors va réagir comme pigment tissulaire , et ainsi provoquer l'apparition de taches sur la peau.

L'invention pallie ces inconvénients. Elle vise une préparation perfectionnée du type en question, qui donne d'excellents résultats sur le plan de la dépigmentation, et qui soit facile à fabriquer, stable, économique, plus efficace, ne présente pas d'effets intempestifs de relargage.

Cette préparation à usage cutané, comprenant des excipients, un vecteur médicamenteux encapsulant du béta-carotène et un mélange de filtres solaires UV-A et UV-B, se caractérise :
- en ce que le vecteur médicamenteux, constitué par des nanosphères encapsulant du béta-carotène, représente de 3 à 6 % du poids de la préparation ;
- en ce que la proportion de filtres UV-B est supérieure à celles des filtres UV-A ;
- et en ce que le filtre UV-B est un dérivé cinnamique.

Par "nanosphères", on désigne des vecteurs médicamenteux formés de particules, généralement sphériques, de taille nanométrique, encapsulant une substance déterminée, en l'occurrence du béta-carotène. Ces composés sont largement décrits dans la littérature.

En d'autres termes, l'invention réside dans une triple sélection, à savoir tout d'abord la sélection d'un vecteur spécifique, à savoir les nanosphères encapsulant du béta-carotène, et ensuite, la sélection d'une proportion de filtres UV-B supérieure à celle des filtres UV-A et enfin, la sélection d'un type précis de filtre UV-B, à savoir les dérivés cinnamiques. On ne pouvait pas penser que cette triple sélection permette de résoudre avec succès les inconvénients des préparations connues jusqu'alors, notamment celles décrites dans le document FR-A-2 591 105 visé dans le préambule, à savoir grâce à la taille réduite des nanosphères, une meilleure pénétration dans la peau et une meilleure biodisponibilité, grâce au rapport des filtres une meilleure efficacité, et enfin, grâce à la nature cinnamique des filtres UV-B une synergie qui possède un effet inhibiteur sur la tyrosinase.

Le choix d'une proportion de filtres UV-B supérieure à celle des filtres UV-A va à l'encontre des enseignements habituels. En effet, on sait que les filtres UV-A sont censés arrêter les radiations UV-A, qui agissent essentiellement sur le derme et au-delà. En revanche, on sait que les filtres UV-B stoppent les radiations UV-B, qui atteignent seulement l'épiderme et s'arrêtent au niveau de la couche basale de l'épiderme. De la sorte, dans une préparation destinée à lutter contre les hyperpigmentations d'origine mélanique, on cherche avant tout à empêcher que les rayons UV atteignent les mélanocytes pour les exciter et ainsi déclencher leur production de mélanine. Or, on sait que les mélanocytes se situent au niveau de la jonction dermo-épidermique, donc dans une zone nettement plus soumise à l'action des rayonnements UV-A que celle des rayonnements UV-B qui atteignent difficilement cette zone. De la sorte, pour protéger les mélanocytes et éviter ou réduire leur excitation par les rayonnements UV, il était d'usage de faire appel à des filtres UV-A, soit seuls, soit en combinaison avec une faible proportion de filtres UV-B.

Or il s'avère que dans le cas d'espèce, pour obtenir une efficacité optimale, on doit utiliser un mélange de filtres UV-A et de filtres UV-B, mais dans lequel la proportion de filtres UV-B est supérieure à celle de filtres UV-A.

Cet effet antinomique et surprenant par rapport aux enseignements antérieurs peut provenir de deux causes.

Tout d'abord, comme les mélanocytes sont essentiellement localisés à la jonction dermo-épidermique, ils sont initialement activés par des rayonnements UV-A. Une fois activés, les organites de ces mélanocytes se mettent à migrer, ce qui les amène dans la zone épidermique entre les kératinocytes où ils sont alors soumis à l'action des rayonnements UV-B qui les excitent et stimulent la production de mélanine. Pour toutes ces raisons, il est impératif d'avoir une proportion de filtres UV-B importante.

Selon une autre caractéristique de l'invention, ces filtres UV-B doivent être des dérivés cinnamiques. Comme dérivés cinnamiques susceptibles d'être utilisés pour cette application, on peut citer :
- le cinnamate de potassium,
- le 4-méthoxycinnamate de propyle,
- le 4-méthoxycinnamate d'amyle ou de 2-ethylcetyl,
- l'acide alphacyano 4 méthoxycinnamique ou ses esters,
- le 4-methoxycinnamate de cyclohexyle,
- l'acide 4 méthoxycinnamique sous forme de ses sels de Potassium, Sodium ou diéthanolamine.

Le composé filtres UV-B préféré est l'octyl-methoxycinnamate.

Avantageusement, en pratique :
- les nanosphères sont des sphères creuses comprenant une membrane en collagène et en glycosaminoglycanne, telles que celles commercialisées par COLETICA sous la marque déposée NANOCOLLASPHERES et NANOTHALASPHERES ; ces nanosphères sont décrites dans le document FR-A- 89 01221 ; ce type de vecteur présente l'avantage, en combinaison avec des excipients et le mélange caractéristique de filtres, de donner des préparations cutanées moins irritantes, totalement biodégradables, biocompatibles et à relargage contrôlé ;
- les nanosphères chargées en béta-carotène représentent de 3 à 6 % du poids de la préparation, de préférence au voisinage de 4 % ; en effet, on a observé que si la proportion de nanosphères est inférieure à 3 %, on n'obtient pratiquement aucun effet sur la pigmentation, et qu'en revanche, si cette proportion excède 6 %, le béta-carotène risque de se comporter en pigment tissulaire ; comme déjà dit, on obtient les meilleurs résultats avec une proportion voisine de 4 % ;
- le mélange de filtres UV-A et de filtres UV-B doit être supérieur à 4 % et inférieur à 15 % du poids de la proportion ; en effet, on a observé que si cette proportion de filtres solaires est inférieure à 4 %, on n'obtient plus d'effet significatif de prévention, et que si cette proportion excède 15 %, on dépasse fréquemment les taux règlementairement admis, avec des risques non négligeables d'irritation cutanée et d'allergies.

La préparation contient également des excipients d'usage courant pour une telle application cutanée. On utilise de préférence des huiles renfermant des acides gras insaturés bien connus pour prévenir le vieillissement de la peau, telles que des huiles de bourrache ou des huiles de germe de blé. Ces excipients ne doivent pas affecter la stabilité des nanoparticules, ni réagir avec les filtres solaires. Il est donc préférable que la proportion de corps gras soit aussi réduite que possible, et notamment inférieure à la moitié, voire au tiers. De manière usuelle, le mélange d'excipients peut contenir aussi des agents anti-oxydants et/ou des agents conservateurs connus pour une telle application.

Dans une forme de réalisation pratique, la préparation selon l'invention comporte :
- 4 % de nanosphères encapsulant du béta-carotène,
- 4 % d'octyl-méthoxycinnamate (filtre UV-B),
- 3 % de butyl méthoxydibenzoylmethane (filtre UV-A),
- 1 % d'agent conservateur,
- 0,1 % d'un agent chélateur d'ions,
- 0,3 % d'un agent gélifiant à base de cellulose modifiée,
- 0,6 % d'un agent destiné à l'ajustement du pH, le reste à 100 % étant constitué par de l'eau déminéralisée et un mélange d'excipients d'usage courant pour une application cutanée.

Il va de soi que dans la préparation selon l'invention, il est indispensable que les nanosphères encapsulant du béta-carotène, les filtres solaires et les excipients ne réagissent pas entre eux. Une fois le mélange de ces composants réalisé, on observe parfois l'apparition d'une coloration orange. On pense que celle-ci peut provenir d'une réaction des filtres UV-A avec les traces de métaux résultant des impuretés contenues dans les autres produits. On pallie cet inconvénient en ajoutant alors un agent chélateur d'ions.

Généralement, la préparation finie est de couleur très claire, voire jaune pâle, selon la qualité des huiles auxquelles on fait appel. Cette préparation est onctueuse, lisse, facile à étaler, se conserve aisément à température ambiante, notamment dans un tube exempt d'air, même au-delà d'une année.

Cette préparation cutanée s'applique aisément à la main, notamment sur le dessus de la main, et pénètre rapidement sous l'effet d'un léger massage.

On obtient de bons résultats en appliquant cette préparation deux fois par jour pendant un à cinq mois, voire de façon continue.

Une fois la préparation mise sur la peau, les filtres solaires arrêtent les radiations UV. Il s'ensuit que les mélanocytes ne sont plus excités et ne fabriquent plus de mélanine, dont les anomalies de production, comme on le sait, peuvent être à l'origine d'hyperpigmentations cutanées.

Simultanément, les nanosphères encapsulant le béta-carotène pénètrent dans le stratum corneum et viennent en contact de la membrane des mélanocytes. Ces deux membranes fusionnent alors, ce qui fait que le béta-carotène passe dans le cytoplasme du mélanocyte où il sature les protéines transportrices spécifiques des caroténoïdes, et/ou les sites récepteurs nucléaires. Il s'ensuit un arrêt de la mélanogénèse, d'où l'arrêt de la production de pigment et par conséquence, l'arrêt de l'apparition de nouvelles taches et de l'intensification des taches existantes. Parallèlement, le dérivé cinnamique choisi par ailleurs pour ses propriétés de filtre UV-B, peut interférer dans la mélanogénèse comme inhibiteur de la tyrosinase, et par ce mécanisme différent de celui précédemment développé, freiner, voire stopper, par son action intrinsèque, la production de mélanine. Grâce à l'élimination naturelle de la mélanine, les taches s'atténuent, puis disparaissent pratiquement totalement dans un délai de trois semaines à cinq mois, ce que l'on ne savait pas obtenir jusqu'alors.

La manière dont l'invention peut être réalisée et les avantages qui en découlent, ressortiront mieux des exemples de réalisation qui suivent.

### Exemple 1 :

A température ambiante, et sous légère agitation, dans 220 grammes (22 %) d'eau déminéralisée, on mélange respectivement :
- 3 grammes (0,30 %) d'un agent gélifiant à base de cellulose modifiée commercialisée par GOODRICH sous la marque "CARBOPOL 934";
- 40 grammes (4 %) de nanosphères encapsulant du béta-carotène, se présentant sous forme d'une suspension aqueuse ayant des concentrations en béta-carotène de 0,5 % commercialisées par COLETICA sous la marque "NANOTHALASPERES", et dont la membrane est en collagène et en glycosaminoglycanne ; ces nanosphères sont présentées comme étant des microcapsules de diamètre inférieur à un (1) micromètre, caractérisées en ce qu'elles comprennent une paroi mixte de collagène marin ou d'atélocollagène, et de glycosaminoglycannes réticulés, la proportion des glycosaminoglycannes par rapport au collagène marin ou à l'atélocollagène pouvant varier de 15 % à 50 % en poids ; les glycosaminoglycannes sont choisis parmi le groupe consistant du chondroïtine-4-sulfate, du chondroïtine-6-sulfate, du dermatane-sulfate, de l'héparine-sulfate, du keratane-sulfate, et de l'héparine et ses dérivés ;
- 10 grammes (1 %) d'un agent conservateur liquide à base de propylèneglycol, de diazolidinyl urée, de méthylparabène et de propylparabène, commercialisé par SUTTON sous la marque "GERMABEN II";
- 1 gramme (0,1 %) d'un agent chélateur d'ions, connu sous le nom d'EDTA dissodique.

On agite lentement à température ambiante, jusqu'à obtenir un mélange bien homogène.

Séparément, sous agitation rapide (1000 tours/minute), et à 80°C, on mélange dans l'ordre :
- 40 grammes (4 %) d'un filtre solaire UV-B à base d'octylméthoxycinnamate, commercialisé par GIVAUDAN sous la marque "PARSOL MCX";
- 30 grammes (3 %) d'un filtre solaire UV-A à base de butylméthoxydibenzoylméthane, commercialisé par GIVAUDAN sous la marque "PARSOL 1789";
- 10 grammes (1 %) d'un excipient à base d'alcool cétylique ;
- 90 grammes (9 %) d'un autre excipient à base de myristate de 2 octyldodécyle ;
- 80 grammes (8 %) d'un excipient à base d'un dérivé non ionique de cire d'abeille blanche, commercialisé par GATTEFOSSE sous la marque "APIFIL" ;
- 10 grammes (1 %) d'un excipient à base d'huile de bourrache ;
- 30 grammes (3 %) d'un excipient à base d'huile de germe de blé ;
- 80 grammes (8 %) d'un excipient à base d'isostéarate d'isostéaryle.

On agite rapidement jusqu'à obtenir une parfaite homogénéisation.

On ajoute alors dans ce dernier mélange de l'eau déminéralisée, chauffée à 82°C à raison de 346,5 grammes (34,65 %). Pour amorcer l'émulsion, on agite rapidement, en versant cette eau dans le mélange.

Lorsque l'émulsion est formée, et que la température est redescendue à 30-35°C, on ajoute alors la première phase contenant les nanosphères de béta-carotène, puis on ajoute 6 grammes (0,6 %) d'une solution à 50 % de triéthanolamine pour ajuster le pH, puis 3 grammes (0,3 %) d'un parfum et enfin 0,5 gramme (0,05 %) d'un agent anti-oxydant commercialisé par GATTEFOSSE sous la référence "WL 774".

On mélange l'ensemble lentement à 30-35°C.

La préparation obtenue est une émulsion de type huile dans eau, de couleur blanche-jaune clair (suivant la qualité des huiles utilisées), lisse, onctueuse, semi-liquide, dont le pH est compris entre 6,8 et 7,5. Son odeur sui-generi, est liée au parfum utilisé.

Cette préparation stable dans le temps (pas de relargage intempestif après un an de stockage), peut être appliquée sur la peau à raison de deux applications par jour pendant une période illimitée.

On obtient, quelquefois dès la troisième semaine, plus généralement dans les cinq mois suivant le début de l'application, une diminution de l'intensité des taches pigmentaires, voire leur disparition totale. On observe une dépigmentation totale entre 7 et 11 semaines dans 75 % des cas traités.

De plus, l'application de cette crème prévient l'apparition de nouvelles taches.

### Exemple 2 :

On répète l'exemple 1 en supprimant les filtres solaires.

En appliquant la préparation cutanée dans les mêmes conditions, on constate que les rayonnements solaires excitent alors les mélanocytes, ce qui entraîne une montée des pigments de la peau.

La disparition des taches est seulement partielle, moins évidente et plus longue.

### Exemple 3 :

On répète l'exemple 1 en supprimant la nanosphère encapsulant le béta-carotène. On observe aucun effet.

### Exemple 4 :

On répète l'exemple 1 en remplaçant les nanosphères par des liposomes du commerce encapsulant du béta-carotène.

On obtient une émulsion ayant sensiblement la même apparence et les mêmes propriétés physiques qu'à l'exemple 1. Toutefois, on observe une dépigmentation totale entre 7 et 11 semaines seulement dans la moitié des cas (contre 75 %).

### Exemple 5 :

On répète l'exemple 1 en inversant la proportion de filtres solaires, à savoir 4 % d'UV-A et 3 % d'UV-B.

La disparition des taches est moins évidente, et obtenue après un temps plus long.

### Exemple 6 :

On répète l'exemple 1 en remplaçant le filtre UV-B de marque "PARSOL MCX" par un autre filtre solaire UV-B à base de 4-méthoxycinnamte de propyle.

On obtient des résultats analogues à ceux obtenus dans l'exemple 1.

Ces exemples montrent bien l'effet de synergie résultant de la combinaison des nanosphères encapsulant du béta-carotène et des filtres solaires UV-A et UV-B.

De la sorte, ces préparations cutanées peuvent être utilisées avec succès sur des patients présentant des hyperpigmentations cutanées d'origine mélanique : taches mélaniques photo-induites, chloasmas, melasmas, pigmentations iatrogènes ou cosmétiques majorées par le soleil, pigmentations des frottements, etc.

## Revendications

1. Préparation à usage cutané, comprenant des excipients, un vecteur médicamenteux encapsulant du béta-carotène et un mélange de filtres solaires UV-A et UV-B, caractérisée :
- en ce que le vecteur médicamenteux, constitué par des nanosphères, représente de 3 à 6 % du poids de la préparation ;
- en ce que la proportion de filtres UV-B est supérieure à celles des filtres UV-A ;
- et en ce que les filtres UV-B présentent une structure cinnamique.

2. Préparation à usage cutané selon la revendication 1, caractérisée en ce que les nanosphères en forme de particules sphériques encapsulant le béta-carotène ont une membrane en collagène et en glycosaminoglycanne.

3. Préparation à usage cutané selon l'une des revendications 1 et 2, caractérisée en ce que les filtres UV-A et UV-B représentent de 4 à 15 % du poids de la préparation.

4. Préparation à usage cutané selon la revendication 1, caractérisée en ce que le filtre UV-B est l'octyl-méthoxycinnamate ou un dérivé cinnamique.

5. Préparation à usage cutané selon l'une des revendications 1 à 4, caractérisée en ce que les excipients contiennent un mélange huiles d'acides gras, des agents anti-oxydants et des agents conservateurs.

6. Préparation à usage cutané comprenant des excipients, des vecteurs médicamenteux encapsulant du béta-carotène et un mélange de filtres UV-A et UV-B, caractérisée en ce qu'elle contient pour cent parties :
- 3 à 6 %, de préférence 4 %, de nanosphères encapsulant du béta-carotène ;
- 4 % d'octyl méthoxycinnamate (filtre UV-B),
- 3 % de butyl méthoxydibenzoylméthane (filtre UV-A),
- 1 % d'agent conservateur,
- 0,1 % d'un agent chelateur d'ions,
- 0,3 % d'un agent gélifiant à base de cellulose modifiée,
- 0,6 % d'un agent pour ajuster le pH,
le reste à 100 % étant constitué par de l'eau déminéralisée et un mélange d'excipients d'usage courant pour une application cutanée.

## Claims

1. Preparation for use on the skin, comprising excipients, a drug carrier encapsulating beta-carotene and a mixture of UV-A and UV-B sunscreen agents, characterized:
- in that the drug carrier, consisting of nanospheres, represents from 3 to 6 % of the weight of the preparation;
- in that the proportion of UV-B screening agents is higher than those of the UV-A screening agents;
- and in that the UV-B screening agents possess a cinnamic structure.

2. Preparation for use on the skin according to Claim 1, chararacterized in that the nanospheres in the form of spherical particles encapsulating beta-carotene have a collagen and glycosaminoglycan membrane.

3. Preparation for use on the skin according to either of Claims 1 and 2, characterized in the UV-A and UV-B screening agents represent from 4 to 15 % of the weight of the preparation.

4. Preparation for use on the skin according to Claim 1, characterized in that the UV-B screening agent is octyl methoxycinnamate or a cinnamic derivative.

5. Preparation for use on the skin according to one of Claims 1 to 4, characterized in that the excipients contain a mixture of fatty acid oils, anti-oxidants and preservatives.

6. Preparation for use on the skin comprising excipients, drug carriers encapsulating beta-carotene and a mixture of UV-A and UV-B screening agents, characterized in that it contains, per one hundred parts:
- 3 to 6 %, and preferably 4 %, of nanospheres encapsulating beta-carotene;
- 4 % of octyl methoxycinnamate (UV-B screening agent);
- 3 % of butylmethoxydibenzoylmethane (UV-A screening agent),
- 1 % of preservative,
- 0.1 % of an ion-chelating agent,
- 0.3 % of a gelling agent based on modified cellulose,
- 0.6 % of an agent for adjusting the pH,
the remainder to 100 % consisting of demineralized water and a mixture of excipients in common use for a cutaneous application.

## Patentansprüche

1. Präparat zur Hautpflege, das Grundmassen, einen β-Carotin einschließenden Arzneimittelträger und eine Mischung von UV-A- und UV-B-Sonnenstrahlungsfiltern enthält, dadurch gekennzeichnet,
- daß der aus Nanokapseln bestehende Arzneimittelträger 3 bis 6 Gewichtsprozent des Präparats ausmacht,
- daß der Anteil UV-B-Filter größer ist als der Anteil UV-A-Filter,
- und daß die UV-B-Filter eine Zimtsäurestruktur aufweisen.

2. Präparat zur Hautpflege nach Anspruch 1, dadurch gekennzeichnet, daß die als kugelförmige Teilchen ausgebildeten Nanokapseln, die das β-Carotin einschließen, eine aus Kollagen und Glykosaminoglykan bestehende Membran aufweisen.

3. Präparat zur Hautpflege nach Anspruch 1 oder 2, dadurch gekennzeichnet, daß die UV-A- und UV-B-Filter 4 bis 15 Gewichtsprozent des Präparats ausmachen.

4. Präparat zur Hautpflege nach Anspruch 1, dadurch gekennzeichnet, daß das UV-B-Filter aus Octyl-Methoxycinnamat oder einem Zimtsäurederivat besteht.

5. Präparat zur Hautpflege nach einem der Ansprüche 1 bis 4, dadurch gekennzeichnet, daß die Grundmassen ein Gemisch von Ölen von Fettsäuren, Antioxydantien und Konservierungsstoffen enthalten.

6. Präparat zur Hautpflege, das Grundmassen, β-Carotin einschließende Arzneimittelträger und eine Mischung von UV-A-Filtern und UV-B-Filtern umfaßt, dadurch gekennzeichnet, daß es folgende prozentualen Anteile enthält:
- 3 bis 6 %, vorzugsweise 4 % β-Carotin einschließende Nanokapseln,
- 4 % Octyl-Methoxycinnamat (UV-B-Filter),
- 3 % Butyl-Methoxydibenzoylmethan (UV-A-Filter),
- 1 % Konservierungsmittel,
- 0,1 % eines Ionen-Chelatbildners,
- 0,3 % eines Gelbildners auf der Basis von modifizierter Cellulose,
- 0,6 % eines Mittels zur Einstellung des pH,
wobei die Ergänzung zu 100 % durch entmineralisiertes Wasser und eine Mischung von üblicherweise für Hautpflegemittel verwendeten Grundmassen gebildet ist.
